(19) Europäisches Patentamt / European Patent Office / Office européen des brevets

(11) **EP 3 108 220 B1**

(12) **EUROPÄISCHE PATENTSCHRIFT**

(45) Veröffentlichungstag und Bekanntmachung des Hinweises auf die Patenterteilung:
**02.05.2018 Patentblatt 2018/18**

(21) Anmeldenummer: **15705585.6**

(22) Anmeldetag: **13.02.2015**

(51) Int Cl.:
$G01N\ 21/31$ (2006.01)   $G01N\ 33/49$ (2006.01)

(86) Internationale Anmeldenummer:
**PCT/EP2015/053131**

(87) Internationale Veröffentlichungsnummer:
**WO 2015/124512 (27.08.2015 Gazette 2015/34)**

(54) **VERFAHREN UND VORRICHTUNG ZUM ZUORDNEN EINER BLUTPLASMAPROBE**

METHOD AND DEVICE FOR ASSIGNING A BLOOD PLASMA SAMPLE

PROCÉDÉ ET DISPOSITIF D'AFFECTATION D'UN ÉCHANTILLON DE PLASMA SANGUIN

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.02.2014 EP 14155821**

(43) Veröffentlichungstag der Anmeldung:
**28.12.2016 Patentblatt 2016/52**

(73) Patentinhaber:
- **Roche Diagnostics GmbH**
  **68305 Mannheim (DE)**
  Benannte Vertragsstaaten:
  **DE**
- **F. Hoffmann-La Roche AG**
  **4070 Basel (CH)**
  Benannte Vertragsstaaten:
  **AL AT BE BG CH CY CZ DK EE ES FI FR GB GR HR HU IE IS IT LI LT LU LV MC MK MT NL NO PL PT RO RS SE SI SK SM TR**

(72) Erfinder:
- **KLINEC, Darko**
  **75365 Calw (DE)**
- **KÖHLER, Michael**
  **71364 Winnenden (DE)**

(74) Vertreter: **Patentanwälte**
**Ruff, Wilhelm, Beier, Dauster & Partner mbB**
**Kronenstraße 30**
**70174 Stuttgart (DE)**

(56) Entgegenhaltungen:
EP-A1- 2 549 264    WO-A1-03/060484
US-A1- 2012 252 127   US-B2- 6 995 835

- **MERRICK M F ET AL: "Evaluation of absorption and first- and second-derivative spectra for simultaneous qualification of bilirubin and hemoglobin", CLINICAL CHEMISTRY, AMERICAN ASSOCIATION FOR CLINICAL CHEMISTRY, WASHINGTON, DC, Bd. 32, Nr. 4, 1. Januar 1986 (1986-01-01) , Seiten 598-602, XP002986171, ISSN: 0009-9147**

**Beschreibung**

Die Erfindung betrifft ein Verfahren und eine Vorrichtung zum Zuordnen einer Blutplasmaprobe zu einer Klasse aus einer vorgegebenen Menge von Klassen, wobei sich die Blutplasmaprobe in einem zumindest teilweise transparenten Gefäß befindet. EP 2 549 264 offenbart Analysegeräte zum Bestimmen der Konzentrationen von Bilirubin, Hämoglobin und Lipiden in einer Körperflüssigkeitsprobe mittels Spektroskopie im Bereich zwischen 340nm und 600nm. Blutproben werden häufig dazu verwendet, bestimmte Krankheiten diagnostizieren zu können oder auch um Straftaten oder Ordnungswidrigkeiten, wie beispielsweise die Einnahme von Drogen oder das Fahren unter Alkoholeinfluss, nachweisen zu können. Eine solche Blutprobe wird typischerweise in ein für Licht transparentes Gefäß in Form eines Kunststoff-Röhrchens eingefüllt, wobei ein solches Röhrchen ähnlich einem Reagenzglas ausgebildet ist. In dem Röhrchen wird die Probe typischerweise vor weiteren Analyseschritten zentrifugiert, wodurch sich ein Blutkuchen am unteren Ende des Röhrchens bildet, in dem alle zellulären Bestandteile der Blutprobe konzentriert sind. Oberhalb des Blutkuchens befindet sich dann typischerweise Blutplasma, das im Wesentlichen die flüssigen Bestandteile der Blutprobe beinhaltet. Das Blutplasma, das eine Blutplasmaprobe bildet, wird in der Regel in nachfolgenden Analyseschritten analysiert.

Häufig ist es sinnvoll, vor weiteren Analyseschritten zumindest eine grobe Bestimmung vorzunehmen, ob die Blutplasmaprobe auf einen besonderen pathologischen Zustand hinweist oder in sonstiger Weise Besonderheiten aufweist. Hierzu kann die Blutplasmaprobe zu einer Klasse aus einer vorgegebenen Menge von Klassen zugeordnet werden, wobei eine solche Zuordnung in der Regel manuell durchgeführt wird. Dies liegt insbesondere daran, dass Blutplasmaproben meistens mit umfangreichen Etiketten versehen sind, welche auf Daten wie den Namen des Patienten oder den Tag der Entnahme hinweisen. Derartige Etiketten verhindern jedoch nach bisheriger Auffassung, dass die Transparenz des Gefäßes dazu ausgenutzt werden kann, mittels optischer Verfahren eine Voranalyse der Blutplasmaprobe durchzuführen.

Typische Klassen, die einer Blutplasmaprobe in dem hier relevanten Stadium zugeordnet werden können, sind beispielsweise eine lipämische Klasse, eine hämolytische Klasse, eine ikterische Klasse und eine gute Klasse. Die gute Klasse oder "gut"-Klasse beinhaltet diejenigen Proben, die nicht der Klasse lipämisch, hämolytisch oder ikterisch zugeordnet sind.

Wenn die Probe der lipämischen Klasse zuzuordnen ist, handelt es sich um eine lipämische Probe, welche einen erhöhten Anteil an Lipiden aufweist. Dies kann beispielsweise ein Hinweis auf eine Störung des Fettstoffwechsels sein.

Wenn die Probe der hämolytischen Klasse zuzuordnen ist, handelt es sich um eine hämolytische Probe, welche einen erhöhten Anteil an Hämoglobin aufweist. Dies kann beispielsweise ein Hinweis auf bestimmte Anämien, Transfusionszwischenfälle oder Malaria sein.

Wenn die Blutplasmaprobe der ikterischen Klasse zuzuordnen ist, handelt es sich um eine ikterische Probe, welche einen erhöhten Anteil an Bilirubin aufweist. Dies kann beispielsweise ein Hinweis auf eine Erkrankung der Leber sein.

Es ist eine Aufgabe der Erfindung, ein Verfahren zum Zuordnen einer Blutplasmaprobe zu einer Klasse aus einer vorgegebenen Menge von Klassen vorzusehen, welches eine automatisierte Zuordnung auch bei etikettierten Gefäßen erlaubt. Es ist des Weiteren eine Aufgabe der Erfindung, eine Vorrichtung vorzusehen, welche ein solches Verfahren ausführt.

Dies wird erfindungsgemäß durch ein Verfahren nach Anspruch 1 und eine Vorrichtung nach Anspruch 11 gelöst. Ausführungsformen der Erfindung können beispielsweise den jeweiligen Unteransprüchen entnommen werden. Der Inhalt der Ansprüche wird durch ausdrückliche Inbezugnahme zum Inhalt der Beschreibung gemacht.

Die Erfindung betrifft ein Verfahren zum Zuordnen einer (Blutplasma-) Probe zu einer Klasse aus einer vorgegebenen Menge von Klassen, wobei sich die Blutplasmaprobe, gegebenenfalls zusammen mit weiteren schichtförmig angeordneten Bestandteilen wie Blutkuchen, Trenngel, usw., in einem zumindest teilweise transparenten Gefäß befindet. Das Verfahren weist folgende Schritte auf: Bestrahlen der Blutplasmaprobe mit Licht, dessen spektrale Zusammensetzung zumindest Wellenlängen aus einer Menge von unterschiedlichen Wellenlängen aufweist, Bilden eines wellenlängenabhängigen Messwerts für eine jeweilige Wellenlänge aus der Menge von Wellenlängen, wobei ein jeweiliger Messwert von einer durch die Blutplasmaprobe und das Gefäß transmittierten Lichtleistung oder transmittierten Lichtintensität bei der jeweiligen Wellenlänge abhängig ist, und Zuordnen der Blutplasmaprobe zu einer Klasse in Abhängigkeit von den wellenlängenspezifischen Messwerten.

Die vorgegebene Menge von Klassen weist eine hämolytische Klasse auf. Die Menge von Wellenlängen weist eine erste hämolytisch-Wellenlänge im Bereich von 535 nm bis 547 nm, bevorzugt 541 nm, und eine zweite hämolytisch-Wellenlänge im Bereich von 510 nm bis 520 nm, bevorzugt 515 nm, auf. Die Blutplasmaprobe wird der hämolytischen Klasse zugeordnet, wenn ein Verhältnis des Messwerts bei der ersten hämolytisch-Wellenlänge und des Messwerts bei der zweiten hämolytisch-Wellenlänge kleiner als ein erster relativer hämolytisch-Schwellenwert ist.

Dieses Vorgehen basiert darauf, dass die Transmission bei einer Wellenlänge von etwa 515 nm nur wenig durch Hämoglobin beeinflusst wird. Somit kann der Messwert bei dieser Wellenlänge als Referenz für den Messwert bei einer Wellenlänge von etwa 541 nm herangezogen werden. Im Gegensatz zum Bestimmen mittels Vergleichens des Absolutwerts des Messwerts mit einem absoluten hämolytisch-Schwellenwert ist es bei dem zuletzt beschriebenen Vergleichen eines Verhältnisses, also eines relativen Werts mit dem relativen hämolytisch-Schwellenwert, nicht erforderlich auszuschließen, dass es sich um eine lipämische Probe handelt, um eine hämolytische Probe zuverlässig bestimmen zu können. Dies liegt daran, dass ein besonders niedriges Verhältnis des Messwerts bei der ersten hämolytisch-Wellenlänge und des Messwerts bei der zweiten hämolytisch-Wellenlänge nur bei hämolytischen Proben auftritt.

Das Verhältnis des Messwerts bei der ersten hämolytisch-Wellenlänge und des Messwerts bei der zweiten hämolytisch-Wellenlänge kann wie folgt berechnet werden:

$$V = M1 / M2,$$

wobei V das Verhältnis der Messwerte, M1 den Messwert bei der ersten hämolytisch-Wellenlänge und M2 den Messwert bei der zweiten hämolytisch-Wellenlänge bezeichnen. M2 wird folglich als Referenz(wert) verwendet.

Es gilt bei der Zuordnung zur hämolytischen Klasse:

$$V < S$$

wobei S den ersten relativen hämolytisch-Schwellenwert bezeichnet.

Der erste relative hämolytisch-Schwellenwert S kann kleiner als 1, bevorzugt kleiner als 0,75, besonders bevorzugt kleiner als 0,5, sein.

Die Erfindung basiert darauf, dass es entgegen der bisher vorherrschenden Meinung möglich ist, mittels transmittierten Lichts eine Zuordnung der Blutplasmaprobe vorzunehmen, und zwar auch dann, wenn sich ein Etikett auf dem Gefäß befindet. Mittels des erfindungsgemäßen Verfahrens ist eine zuverlässige und schnelle automatische Überprüfung von Blutplasmaproben möglich, was insbesondere die Verwendung des Verfahrens in einem auf hohen Durchsatz ausgelegten Analysesystem erlaubt.

Beim Bilden eines wellenlängenspezifischen Messwerts kann entweder genau ein wellenlängenspezifischer Messwert gebildet werden oder es können auch mehrere wellenlängenspezifische Messwerte bei einer identischen Wellenlängen gebildet werden, wobei beispielsweise ein Mittelwert über die Messwerte gebildet wird.

Der jeweilige Messwert kann von einer durch die Blutplasmaprobe und das Gefäß geradlinig transmittierten Lichtleistung abhängig sein. Damit wird ermittelt, welcher Anteil des auf einer Seite abgegebenen Lichts durch die Probe geradlinig durchtritt und welcher Anteil innerhalb der Probe absorbiert oder auch gestreut wird. Diese Information kann zum Zuordnen der Blutplasmaprobe zu einer Klasse verwendet werden.

Gemäß einer Ausführung weist die vorgegebene Menge von Klassen eine lipämische Klasse auf. Die Menge von Wellenlängen weist eine lipämisch-Wellenlänge im Bereich von 610 nm bis 700 nm, bevorzugt 650 nm oder 685 nm, auf. Die Blutplasmaprobe wird der lipämischen Klasse zugeordnet, wenn der Messwert bei der lipämisch-Wellenlänge kleiner als ein lipämisch-Schwellenwert ist.

Diese Ausführung basiert darauf, dass Lipide im Bereich von 610 nm bis 700 nm, und insbesondere bei 650 nm sowie bei 685 nm, die Transmission durch die Blutplasmaprobe besonders stark herabsetzen. Somit kann durch Vergleichen des entsprechenden Messwerts mit einem geeignet gewählten lipämisch-Schwellenwert eine lipämische Probe erkannt werden.

Gemäß einer Ausführung weist die vorgegebene Menge von Klassen eine lipämische Klasse auf. Ein Streuungs-Messwert wird gebildet, der von einer von der Blutplasmaprobe gestreuten Lichtleistung oder gestreuten Lichtintensität abhängig ist, wobei die Blutplasmaprobe der lipämischen Klasse zugeordnet wird, wenn der Streuungs-Messwert größer als ein lipämisch-Streuungsschwellenwert ist. Diese Streuungsmessung kann mit der Transmissionsmessung kombiniert werden. Der Streuungs-Messwert kann von einer seitlich, beispielsweise unter einem Winkel von 90° zur geradlinigen Transmission, gestreuten Lichtleistung abhängig sein. Dies kann beispielsweise durch Anordnung eines entsprechenden Detektors erreicht werden.

Der Streuungs-Messwert kann von einer elastisch gestreuten Lichtleistung oder von einer elastisch gestreuten Lichtintensität abhängig sein. Elastisch bezeichnet in diesem Zusammenhang die Tatsache, dass die lipämisch-Wellenlänge konstant über den Streuungsprozess bleibt.

Die Erkennung einer lipämischen Probe anhand eines solchen Streuungs-Messwerts basiert auf der Erkenntnis, dass Lipide gewisse Wellenlängen besonders stark streuen. Dies trifft für keine der anderen hier relevanten zu ermittelnden Stoffe in der Blutplasmaprobe zu. Somit eignet sich die Verwendung eines Streuungs-Messwerts in besonderer Weise für die Erkennung einer lipämischen Probe.

Gemäß einer Ausführung wird die Blutplasmaprobe der hämolytischen Klasse zugeordnet, wenn der Messwert bei der ersten hämolytisch-Wellenlänge kleiner als ein absoluter hämolytisch-Schwellenwert ist und wenn die Blutplasmaprobe keiner lipämischen Klasse zugeordnet wird.

Diese Ausführung basiert darauf, dass die Transmission bei einer hämoglobinhaltigen Probe bei einer Wellenlänge von etwa 541 nm besonders niedrig ist, was grundsätzlich zur Erkennung einer hämolytischen Probe verwendet werden kann. Bei einem hohen Gehalt von Lipiden weist eine lipämische Probe bei der genannten Wellenlänge jedoch ebenfalls nur eine niedrige Transmission auf. Daher wäre eine alleinige Betrachtung des Messwerts bei einer Wellenlänge von etwa 541 nm noch nicht ausreichend, um eine hämolytische Probe zuverlässig zu erkennen. Deshalb kann die Blutplasmaprobe nur dann basierend auf einem Vergleich des Absolutwerts des Messwerts mit dem absoluten hämolytisch-Schwellenwert der hämolytischen Klasse zugeordnet werden, wenn sichergestellt ist, dass es sich nicht um eine lipämische Probe handelt. Da eine lipämische Probe, wie weiter oben beschrieben, bei deutlich höheren Wellenlängen oder mittels Streulicht zuverlässig erkannt werden kann, ist es auf diese Weise möglich, hämolytische, lipämische und gute Proben zuverlässig voneinander zu unterscheiden. Es sei erwähnt, dass die Feststellung, dass es sich nicht um eine lipämische Probe handelt, nicht zwingend auf einem erfindungsgemäßen Verfahrensschritt und nicht zwingend auf einer optischen Messung basieren muss. Es kann beispielsweise auch durch externe Faktoren sichergestellt sein, dass lipämische Proben nicht bis zur Ausführung des Verfahrens gelangen, z.B. durch ein vorgeschaltetes Sortiergerät oder weil Proben nur von einer bestimmten Krankenstation kommen.

Gemäß einer Ausführung weist die Menge von Wellenlängen eine dritte hämolytisch-Wellenlänge im Bereich von 610 nm bis 700 nm, bevorzugt 650 nm oder 685 nm, auf. Die Blutplasmaprobe wird der hämolytischen Klasse zugeordnet, wenn ein Verhältnis des Messwerts bei der ersten hämolytisch-Wellenlänge und des Messwerts bei der dritten hämolytisch-Wellenlänge kleiner als ein zweiter relativer hämolytisch-Schwellenwert ist.

Diese Ausführung basiert darauf, dass auch ein Messwert bei einer Wellenlänge von 610 bis 700 nm, bevorzugt 650 nm oder 685 nm, anstelle der vorher beschriebenen Wellenlänge von etwa 515 nm, als Referenzwert verwendet werden kann. Ein besonders niedriger Wert des beschriebenen Verhältnisses des Messwerts bei der ersten hämolytisch-Wellenlänge und des Messwerts bei der dritten Hämolytisch-Wellenlänge tritt lediglich bei einer hämolytischen Probe auf, so dass dieses Verhältnis auch für sich genommen zuverlässig zur Erkennung einer hämolytischen Probe verwendet werden kann. Es sei angemerkt, dass bei einer lipämischen Probe - wie weiter oben beschrieben wurde - der Messwert bei der dritten hämolytisch-Wellenlänge besonders niedrig ist, wodurch das hier beschriebene Verhältnis erhöht werden würde.

Das Verhältnis des Messwerts bei der ersten hämolytisch-Wellenlänge und des Messwerts bei der dritten hämolytisch-Wellenlänge kann wie folgt berechnet werden:

$$V2 = M1 / M3,$$

wobei V2 das Verhältnis der Messwerte, M1 den Messwert bei der ersten hämolytisch-Wellenlänge und M3 den Messwert bei der dritten hämolytisch-Wellenlänge bezeichnen. M3 wird folglich als Referenz(wert) verwendet.

Es gilt bei der Zuordnung zur hämolytischen Klasse:

$$V2 < S2$$

wobei S2 den zweiten relativen hämolytisch-Schwellenwert bezeichnet.

Der zweite relative hämolytisch-Schwellenwert S2 kann kleiner als 1, bevorzugt kleiner als 0,25, besonders bevorzugt kleiner als 0,1, sein.

Gemäß einer Ausführung weist die vorgegebene Menge von Klassen eine ikterische Klasse auf. Die Menge von Wellenlängen weist eine erste ikterisch-Wellenlänge im Bereich von 450 nm bis 485 nm, bevorzugt 470 nm, auf. Die Blutplasmaprobe wird der ikterischen Klasse zugeordnet, wenn der Messwert bei der ersten ikterisch-Wellenlänge kleiner als ein absoluter ikterisch-Schwellenwert ist und wenn die Blutplasmaprobe keiner lipämischen Klasse zugeordnet wird und keiner hämolytischen Klasse zugeordnet wird.

Dieses Vorgehen basiert darauf, dass eine besonders niedrige Transmission im Bereich von 450 nm bis 485 nm, insbesondere bei 470 nm, bei ikterischen Proben aufgrund des enthaltenen Bilirubins auftritt. Eine niedrige Transmission in diesem Wellenlängenbereich kann jedoch auch bei lipämischen Proben oder bei hämolytischen Proben auftreten, insbesondere bei einer besonders hohen Konzentration von Lipiden oder von Hämoglobin. Deshalb ist es bevorzugt, eine Blutplasmaprobe nur dann aufgrund eines Vergleichens des Absolutwerts des Messwerts bei der ersten ikterisch-Wellenlänge mit dem absoluten ikterisch-Schwellenwert der ikterischen Klasse zuzuordnen, wenn ausgeschlossen werden kann, dass es sich um eine lipämische oder um eine hämolytische Probe handelt. Dies kann, wie weiter oben beschrieben wurde, außerhalb des Bereichs von 450 nm bis 485 nm erfolgen. Auf diese Weise können ikterische, hämolytische, lipämische und gute Proben zuverlässig voneinander unterschieden werden.

Auch in diesem Fall ist es möglich, durch externe Maßnahmen, wie weiter oben beschrieben wurde, auszuschließen, dass es sich um eine lipämische oder um eine hämolytische Probe handelt.

Gemäß einer Ausführung weist die vorgegebene Menge von Klassen eine ikterische Klasse auf. Die Menge von Wellenlänge weist eine erste ikterisch-Wellenlänge im Bereich von 450 nm bis 485 nm, bevorzugt 470 nm, auf und weist eine zweite ikterisch-Wellenlänge im Bereich von 510 nm bis 520 nm, bevorzugt 515 nm, auf. Die Blutplasmaprobe wird der ikterischen Klasse zugeordnet, wenn ein Verhältnis des Messwerts bei der ersten ikterisch-Wellenlänge und des Messwerts bei der zweiten ikterisch-Wellenlänge kleiner als ein erster relativer ikterisch-Schwellenwert ist. Der erste relative ikterisch-Schwellenwert kann beispielsweise 0,1 betragen.

Dieses Vorgehen basiert darauf, dass die Transmission durch eine Blutplasmaprobe im Bereich von 510 nm bis 520 nm und insbesondere bei 515 nm als Referenz für die Bestimmung einer ikterischen Probe verwendet werden kann, ebenso wie dies bereits weiter oben mit Bezug auf eine hämolytische Probe erläutert wurde. Auf eine separate Absicherung, dass es sich nicht um eine hämolytische oder lipämische Probe handelt, kann verzichtet werden, da ein besonders niedriges Verhältnis des Messwerts bei der ersten ikterisch-Wellenlänge und des Messwerts bei der zweiten ikterisch-Wellenlänge nur bei einer ikterischen Probe auftritt.

Die zweite ikterisch-Wellenlänge kann gleich der zweiten hämolytisch-Wellenlänge sein. Dies ist dann günstig, wenn die vorgegebene Menge von Klassen sowohl eine ikterische Klasse wie auch eine hämolytische Klasse aufweist, da dann eine Wellenlänge für die Zuordnung zu beiden Klassen verwendet werden kann. Damit wird ein apparativer Aufwand reduziert.

Gemäß einer Ausführung weist die vorgegebene Menge von Klassen eine ikterische Klasse auf. Die Menge von Wellenlängen weist eine erste ikterisch-Wellenlänge im Bereich von 450 nm bis 485 nm, bevorzugt 470 nm, auf und weist eine dritte ikterisch-Wellenlänge im Bereich von 610 nm bis 700 nm, bevorzugt 650 nm oder 685 nm, auf. Die Blutplasmaprobe wird der ikterischen Klasse zugeordnet, wenn ein Verhältnis des Messwerts bei der ersten ikterisch-Wellenlänge und des Messwerts bei der dritten ikterisch-Wellenlänge kleiner als ein zweiter relativer ikterisch-Schwellenwert ist. Der zweite relative ikterisch-Schwellenwert kann beispielsweise 0,1 betragen.

Dieses Vorgehen basiert darauf, dass der Wellenlängenbereich von 610 nm bis 700 nm, und insbesondere 650 nm oder 685 nm, ebenso als Referenz für die Bestimmung einer ikterischen Probe verwendet werden kann wie der weiter oben beschriebene Wellenlängenbereich von 510 nm bis 520 nm. Dies beruht darauf, dass bei einer ikterischen Probe der Messwert in diesem Wellenlängenbereich nicht oder nur wenig verringert ist.

Die dritte ikterisch-Wellenlänge kann gleich der dritten hämolytisch-Wellenlänge sein. Dies ist insbesondere dann günstig, wenn die Menge von Klassen sowohl eine ikterische Klasse wie auch eine hämolytische Klasse aufweist. Wie bereits weiter oben beschrieben wurde, kann durch ein solches Vorgehen ein apparativer Aufwand reduziert werden.

Gemäß einer Ausführung weist die vorgegebene Menge von Klassen eine gute Klasse auf. Die Blutplasmaprobe wird der guten Klasse zugeordnet, wenn die Blutplasmaprobe keiner lipämischen Klasse zugeordnet wird, und keiner hämolytischen Klasse zugeordnet wird und keiner ikterischen Klasse zugeordnet wird.

Das beschriebene Vorgehen kann bedeuten, dass bei einer Probe überprüft wird, ob es sich um eine lipämische Probe handelt, ob es sich um eine hämolytische Probe handelt und ob es sich um eine ikterische Probe handelt. Erst wenn festgestellt wird, dass es sich weder um eine lipämische noch um eine hämolytische noch um eine ikterische Probe handelt, wird die Blutplasmaprobe der guten Klasse zugeordnet. Es kann jedoch auch bedeuten, dass eine oder zwei der beschriebenen Klassen nicht überprüft werden und somit die Blutplasmaprobe bereits dann der guten Klasse zugeordnet wird, wenn festgestellt wird, dass es sich nicht um eine der überprüften Klassen von Proben handelt. Dies kann beispielsweise dann günstig sein, wenn aufgrund von Einflussfaktoren, welche außerhalb eines für die Durchführung verwendeten Untersuchungssystems liegen, also z.B. durch externe Maßnahmen, sichergestellt oder es zumindest unwahrscheinlich ist, dass eine bestimmte Klasse von Proben auftritt oder dass sogar zwei bestimmte Klassen von Proben auftreten.

Gemäß einer Ausführung wird eine Fehlermeldung ausgegeben, wenn die Blutplasmaprobe zumindest zwei unterschiedlichen Klassen zugeordnet wird. Eine Fehlermeldung kann beispielsweise ein akustischer Warnton und/oder eine Fehleranzeige auf einem Display sein. Damit wird der Tatsache Rechnung getragen, dass es für diesen Fall wahrscheinlich ist, dass zumindest ein Erkennungsverfahren für eine bestimmte Klasse von Proben versagt hat und eine manuelle Bestimmung der Klasse der Blutplasmaprobe oder ein Abbruch weiterer Analyseschritte ratsam ist.

Gemäß einer bevorzugten Ausführung weist das Verfahren einen Schritt auf, in welchem erkannt wird, ob und bevorzugt auch in welcher Lage sich ein Etikett auf dem Gefäß befindet, wobei abhängig davon eine Anzahl von Schwellenwerten verändert wird. Unter Umständen kann auch ein Drehen der Probe derart erfolgen, dass das transmittierte Licht möglichst wenig Etikettenschichten passieren muss.

Damit wird der Tatsache Rechnung getragen, dass sich die Transmission bei allen Wellenlängen verändert, wenn ein zur Messung verwendeter Lichtstrahl durch dieses Etikett hindurchtritt. Dabei kann auch unterschieden werden, ob der Lichtstrahl nur beim Eintritt und bei Austritt oder sowohl beim Eintritt wie auch beim Austritt durch das Etikett hindurchtritt. Durch die Anpassung von Schwellenwerten abhängig von einer solchen Erkennung kann die Zuverlässigkeit von Verfahrensschritten, welche basierend auf dem Vergleichen mit einem solchen Schwellenwert eine Zuordnung der Probe zu einer Klasse vornehmen, erhöht werden.

Grundsätzlich können alle verwendeten Schwellenwerte verändert werden. Es kann auch eine Untergruppe der verwendeten Schwellenwerte verändert werden. Insbesondere können die weiter oben beschriebenen Schwellenwerte verändert werden.

Es kann sinnvoll sein, die absoluten Schwellenwerte zu verändern, da bei einem Vergleich von Absolutwerten keine Korrektur durch einen Referenzwert, welcher ebenfalls dem Einfluss des Etiketts unterliegen würde, vorhanden ist. Somit kann/können ein Schwellenwert oder mehrere Schwellenwerte aus der Gruppe von Schwellenwerten, welche aus dem absoluten lipämisch-Schwellenwert, dem lipämisch-Streuungsschwellenwert, dem absoluten hämolytisch-Schwellenwert und dem absoluten ikterisch-Schwellenwert besteht, verändert werden. Ebenso können jedoch auch die relativen Schwellenwerte verändert werden, also beispielsweise ein Schwellenwert oder mehrere Schwellenwerte aus der Gruppe von Schwellenwerten, welche aus dem ersten relativen hämolytisch-Schwellenwert, dem zweiten relativen hämolytisch-Schwellenwert, dem ersten relativen ikterisch-Schwellenwert und dem zweiten relativen ikterisch-Schwellenwert besteht.

Gemäß einer Weiterbildung kann auch erkannt werden, ob der Lichtstrahl durch mehrere Etiketten, also nicht nur durch ein Etikett, hindurchtritt. Auch in Abhängigkeit davon können die erwähnten Schwellenwerte in geeigneter Weise verändert werden.

Die beschriebene Veränderung von Schwellenwerten ist insbesondere dann besonders vorteilhaft, wenn eine verhältnismäßig geringe Lichtleistung verwendet wird. Bei hohen Lichtleistungen kann der Einfluss von Etiketten auch ausreichend gering sein, so dass auf die Veränderung von Schwellenwerten verzichtet werden kann.

Der Schritt des Erkennens, ob und bevorzugt auch in welcher Lage sich ein Etikett auf dem Gefäß befindet, wird bevorzugt mittels einer Kamera ausgeführt. Es können jedoch auch andere Messinstrumente, wie beispielsweise ein Fotodetektor, verwendet werden.

Die Erfindung betrifft des Weiteren eine Vorrichtung zum Zuordnen einer Blutplasmaprobe, welche sich in einem zumindest teilweise transparenten Gefäß befindet, unter Verwendung des oben genannten Verfahrens. Die Vorrichtung weist auf: eine Lichtquelle, die Licht, dessen spektrale Zusammensetzung zumindest eine Wellenlänge aus der gemäß dem Verfahren vorgegebenen Menge von Wellenlängen aufweist, auf das Gefäß abgibt, eine Detektoranordnung, die durch die Blutplasmaprobe und das Gefäß (geradlinig) transmittiertes Licht aufnimmt und darin für jede der Wellenlängen aus der Menge von Wellenlängen einen von der transmittierten Lichtleistung oder der transmittierten Lichtintensität abhängigen Messwert ermittelt, und eine Steuerungsvorrichtung, die mit der Detektoranordnung zur Erfassung der gemessenen Messwerte verbunden ist und dazu ausgebildet ist, das erfindungsgemäße Verfahren auszuführen. Die Steuerungsvorrichtung kann beispielsweise einer herkömmlicher Personal Computer sein, auf dem ein Programm abläuft, mittels dem das erfindungsgemäße Verfahren ausgeführt wird.

Mit einer solchen Vorrichtung kann das erfindungsgemäße Verfahren durchgeführt werden. Die Vorrichtung ermöglicht somit die Erkennung der Klasse einer Blutplasmaprobe oder anders ausgedrückt die Zuordnung einer Blutplasmaprobe zu einer Klasse, ohne dass hierfür ein manueller Eingriff notwendig wäre, und zwar auch dann, wenn sich auf dem Gefäß ein Etikett befindet.

Bei der Durchführung des erfindungsgemäßen Verfahrens mit der erfindungsgemäßen Vorrichtung kann auf alle weiter oben beschriebenen Ausgestaltungen des Verfahrens in beliebiger Kombination zurückgegriffen werden. Erläuterte Vorteile gelten entsprechend.

Gemäß einer Ausführung weist die Detektoranordnung für jede Wellenlänge aus der Menge von Wellenlängen einen Detektor, einen dem Detektor vorgeschalteten Filter und eine lichtleitende Faser zur Aufnahme von durch die Probe und das Gefäß transmittiertem Licht auf.

Bei einem Detektor kann es sich beispielsweise um einen üblichen Fotodetektor, wie beispielsweise einen Halbleiterdetektor, handeln.

Bei dem Filter kann es sich beispielsweise um einen optischen Filter oder um einen Interferenzfilter handeln. Ein solcher Filter hat üblicherweise nur einen schmalen Transmissionsbereich, so dass ein Wellenlängenbereich von nur einem oder nur wenigen Nanometern schmalbandig durch den Filter transmittiert wird. Solche Filter können auch als Bandpassfilter bezeichnet werden. Damit kann festgelegt werden, von welcher Wellenlänge bzw. von welchem Wellenlängenbereich der nachgeschaltete Detektor einen Messwert aufnimmt.

Die Verwendung von lichtleitenden Fasern ermöglicht eine zuverlässige Lichtleitung zu einem Ort, an welchem sich ein Detektor befindet, was die Freiheit bei der Anordnung der Detektoren erhöht. Außerdem können damit problemlos mehrere Detektoren verwendet werden, was beim Zwang zur Anbringung von Detektoren unmittelbar an dem Gefäß problematisch sein könnte.

Die Vorrichtung kann eine weitere Detektoranordnung aufweisen, die von der Blutplasmaprobe relativ zur geradlinigen Ausbreitung seitlich gestreutes Licht, bevorzugt mit einem Winkel von 90°, aufnimmt und einen von der gestreuten Lichtleistung oder der gestreuten Lichtintensität abhängigen weiteren Messwert ermittelt, wobei die Steuerungsvorrichtung mit der weiteren Detektoranordnung zur Erfassung des weiteren Messwerts verbunden ist.

Damit kann ein Verfahren ausgeführt werden, welches auf der Messung von Streulicht basiert, wie weiter oben mit Bezug auf die Zuordnung einer Blutplasmaprobe zur lipämischen Klasse erläutert wurde.

Bei der Steuerungsvorrichtung kann es sich beispielsweise um einen üblichen Computer oder um eine andere elektronische Vorrichtung mit einem Prozessor, einem Speichermedium und geeigneten Schnittstellen handeln.

Die Vorrichtung kann eine Kamera aufweisen, mit welcher Etiketten auf dem Gefäß erkannt werden können. Hierzu können geeignete und bekannte Bilderkennungsalgorithmen verwendet werden. Dies ist besonders geeignet zur oben beschriebenen Variation von Schwellenwerten in Abhängigkeit davon, ob ein Lichtstrahl durch ein Etikett oder durch mehrere Etiketten hindurchtritt.

Die Erfindung wird nachfolgend unter Bezugnahme auf die Zeichnungen detailliert beschrieben. Hierbei zeigen schematisch:

Fig. 1:    typische Transmissionsspektren im sichtbaren Wellenlängenbereich bei unterschiedlichen Klassen von Proben,

Fig. 2:    eine Vorrichtung zum Zuordnen einer Blutplasmaprobe, die ein Verfahren zum Zuordnen einer Blutplasmaprobe zu einer Klasse ausführt.

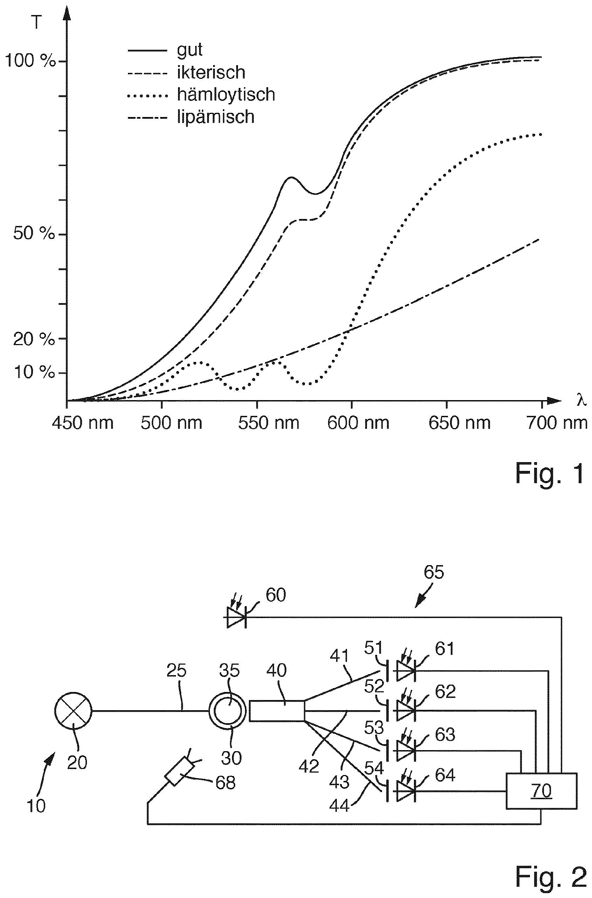

Fig. 1 zeigt typische, schematisch dargestellte Transmissionsspektren von guten, ikterischen, hämolytischen und lipämischen Proben. Auf der horizontalen Achse ist dabei die Wellenlänge ($\lambda$) im Bereich von 450 nm bis 700 nm, also etwa im sichtbaren Spektrum, dargestellt. Auf der vertikalen Achse ist die Transmission (T) in Werten von 0 % bis 100 % dargestellt.

Die durchgezogene Kurve zeigt ein typisches Transmissionsspektrum einer guten Probe. Es ist dabei erkennbar, dass der Wert der Transmission von etwa 0 % bis 100 % über den gezeigten Wellenlängenbereich von 450 nm bis 700 nm ansteigt. Zwischen 550 nm und 600 nm ist die Kurve strukturiert, und zwar mit einem Maximum und einem Minimum.

Die gestrichelte Kurve zeigt ein typisches Transmissionsspektrum einer ikterischen Probe. Es ist erkennbar, dass im Bereich unterhalb von etwa 600 nm die Transmission unterhalb derjenigen einer guten Probe liegt. Dies kann zur Erkennung einer ikterischen Probe verwendet werden. Insbesondere ist der Wert der Transmission bei einer Wellenlänge von 470 nm kleiner als bei einer guten Probe.

Die gepunktete Kurve zeigt ein typisches Transmissionsspektrum einer hämolytischen Probe. Dabei ist zu erkennen, dass die Transmission bei einer solchen hämolytischen Probe grundsätzlich unterhalb der Transmission einer guten Probe liegt. Des Weiteren weist eine solche hämolytische Probe bei einer Wellenlänge von 541 nm (und ebenfalls bei ca. 580 nm) ein zusätzliches Minimum in der Transmissionskurve auf. Dies kann zur Erkennung einer solchen hämolytischen Probe verwendet werden.

Die in Strich-Punkt-Folge dargestellte Kurve zeigt ein typisches Transmissionsspektrum einer lipämischen Probe. Dabei ist zu erkennen, dass die Transmission bei einer solchen lipämischen Probe grundsätzlich unterhalb der Transmission einer guten Probe liegt. Insbesondere gilt dies für den Bereich von 610 nm bis 700 nm, was für die Erkennung einer solchen lipämischen Probe verwendet werden kann.

Fig. 2 zeigt eine Vorrichtung 10 zum Zuordnen einer Blutplasmaprobe zu einer Klasse aus einer vorgegebenen Menge von Klassen.

Die Vorrichtung 10 weist eine Lichtquelle in Form einer Halogenlampe 20 auf, welche einen Lichtstrahl 25 mit breitbandigem Spektrum aussendet. Der Begriff Lichtstrahl umfasst auch Lichtschnitte.

Die Vorrichtung 10 weist einen Probenhalter 30 auf, in welchem ein zumindest teilweise transparentes Gefäß 35 aufgenommen werden kann. In dem Gefäß 35 befindet sich eine zentrifugierte Blutprobe bzw. Blutplasmaprobe.

Der Lichtstrahl 25 trifft auf das Gefäß 35 und tritt durch das Gefäß 35 hindurch. Das Gefäß 35 ist derart zur Lichtquelle 20 ausgerichtet, dass der Lichtstrahl 25 durch das Blutplasma hindurchtritt.

Gegenüberliegend zur Halogenlampe 20 ist angrenzend an den Probenhalter 30 ein Lichtsammler 40 vorgesehen, welcher denjenigen Teil des Lichtstrahls 25 aufnimmt, welcher durch das Gefäß 35, und damit auch durch die Probe, transmittiert wird. Von dem Lichtsammler 40 gehen lichtleitende Fasern in Form eines ersten Glasfaserkabels 41, eines zweiten Glasfaserkabels 42, eines dritten Glasfaserkabels 43 und eines vierten Glasfaserkabels 44 ab.

Das erste Glasfaserkabel 41 führt zu einem ersten Bandpassfilter 51, hinter welchem sich eine erste Fotodiode 61 befindet. Das zweite Glasfaserkabel 42 führt zu einem zweiten Bandpassfilter 52, hinter welchem sich eine zweite Fotodiode 62 befindet. Das dritte Glasfaserkabel 43 führt zu einem dritten Bandpassfilter 53, hinter welchem sich eine dritte Fotodiode 63 befindet. Das vierte Glasfaserkabel 44 führt zu einem vierten Bandpassfilter 54, hinter welchem sich eine vierte Fotodiode 64 befindet.

Außerdem ist noch eine weitere Fotodiode 60 seitlich zum Verlauf des Lichtstrahls 25 angeordnet, die gestreutes Licht detektiert. Zusammen bilden der Lichtsammler 40, die Glasfaserkabel 41, 42, 43, 44 die Bandpassfilter 51, 52, 53, 54 die weitere Fotodiode 60 und die erste, zweite, dritte und vierte Fotodiode 61, 62, 63, 64 eine Detektoranordnung 65. Es versteht sich, dass weitere Glasfaserkabel und zugehörige Fotodioden für entsprechende, weitere Wellenlängen vorgesehen sein können.

Die Vorrichtung 10 weist des Weiteren eine Kamera 68 auf, welche das Gefäß 35 in dem Probenhalter 30 erfasst. Damit kann beispielsweise ein Etikett und eine Lage des Etiketts auf dem Gefäß 35 erkannt werden.

Die Fotodioden 60, 61, 62, 63, 64 und die Kamera 68 sind mit einer elektronischen Steuerungsvorrichtung 70 verbunden. Diese nimmt Signale der Fotodioden 60, 61, 62, 63, 64 und der Kamera 68 auf, um mittels eines erfindungsgemäßen Verfahrens die in dem Gefäß 35 enthaltene Probe zu einer Klasse aus einer Menge von vorgegebenen Klassen zuzuordnen. Bei diesen Klassen handelt es sich um eine lipämische Klasse, eine hämolytische Klasse, eine ikterische Klasse und eine gute Klasse.

Der erste Bandpassfilter 51 weist ein Transmissionsmaximum bei 685 nm auf, was einer lipämisch-Wellenlänge entspricht. Sofern ein von der ersten Fotodiode 61 ermittelter Messwert kleiner ist als ein vorgegebener lipämisch-Schwellenwert, ordnet die elektronische Steuerungsvorrichtung 70 die Probe der lipämischen Klasse zu. Des Weiteren wird die Probe ebenfalls der lipämischen Klasse zugeordnet, wenn ein Messwert der gestreutes Licht detektierenden weiteren Fotodiode 60 größer ist als ein Lipämisch-Streuungsschwellenwert. Sollten beide beschriebenen Möglichkeiten zur Zuordnung der Probe zur lipämischen Klasse zu unterschiedlichen Ergebnissen führen, so wird eine Fehlermeldung ausgegeben.

Der zweite Bandpassfilter 52 weist ein Transmissionsmaximum bei 541 nm auf, was einer ersten hämolytisch-Wellenlänge entspricht. Sofern ein von der zweiten Fotodiode 62 detektierter Messwert kleiner ist als ein vorgegebener absoluter hämolytisch-Schwellenwert, und wenn gleichzeitig die Probe nicht der lipämischen Klasse zugeordnet wurde, ordnet die elektronische Steuerungsvorrichtung 70 die Probe der hämolytischen Klasse zu. Des Weiteren berechnet die elektronische Steuerungsvorrichtung 70 auch ein Verhältnis der Messwerte, welche von dem zweiten Detektor 62 und dem ersten Detektor 61 gemessen werden. Sofern dieses Verhältnis geringer ist als ein relativer hämolytisch-Schwellenwert, ordnet die elektronische Steuerungsvorrichtung 70 die Probe ebenfalls der hämolytischen Klasse zu. Sollten beide beschriebenen Möglichkeiten zur Zuordnung der Probe zur hämolytischen Klasse zu unterschiedlichen Ergebnissen führen, wird eine Fehlermeldung ausgegeben.

Der dritte Bandpassfilter 53 weist ein Transmissionsmaximum bei 515 nm auf, was einer zweiten ikterisch-Wellenlänge entspricht. Der vierte Bandpassfilter 54 weist ein Transmissionsmaximum bei 470 nm auf, was einer ersten ikterisch-Wellenlänge entspricht. Sofern ein von der vierten Fotodiode 64 gemessener Messwert kleiner ist als ein absoluter ikterisch-Schwellenwert und wenn gleichzeitig die Blutplasmaprobe weder der lipämischen Klasse noch der hämolytischen Klasse zugeordnet wird, ordnet die elektronische Steuerungsvorrichtung 70 die Probe der ikterischen Klasse zu. Des Weiteren berechnet die elektronische Steuerungsvorrichtung 70 auch ein Verhältnis der Messwerte, welche von dem vierten Detektor 64 und dem dritten Detektor 63 gemessen werden. Sofern dieses Verhältnis kleiner ist als ein relativer ikterisch-Schwellenwert, ordnet die elektronische Steuerungsvorrichtung 70 die Probe ebenfalls der ikterischen Klasse zu. Sollten beide beschriebenen Möglichkeiten zur Zuordnung der Probe zur ikterischen Klasse zu unterschiedlichen Ergebnissen führen, wird eine Fehlermeldung ausgegeben.

Sofern die Steuerungsvorrichtung 70 die Probe weder der lipämischen Klasse, noch der hämolytischen Klasse, noch der ikterischen Klasse zuordnet, ordnet sie die Probe der guten Klasse zu.

Mittels eines von der Kamera 68 gelieferten Bilds ermittelt die elektronische Steuerungsvorrichtung 70, ob der Lichtstrahl 25 auf seinem Weg durch das Gefäß 35 und die darin enthaltene Probe bzw. das darin enthaltene Blutplasma ungehindert verlaufen kann, oder ob er durch eines oder mehrere Etiketten hindurchtreten muss. Unter einem ungehinderten Verlauf wird insbesondere verstanden, dass der Lichtstrahl 25 lediglich durch die Probe bzw. das darin enthaltene Blutplasma und durch transparente Teile des Gefäßes 35 hindurchtritt. In Abhängigkeit davon verändert die elektronische Steuerungsvorrichtung 70 die erwähnten Schwellenwerte in vorgegebener Art und Weise. Insbesondere werden dann, wenn erkannt wurde, dass der Lichtstrahl 25 durch zumindest ein Etikett hindurchtreten muss, die absoluten Schwellenwerte verringert, da der Lichtstrahl in diesem Fall teilweise vom Etikett absorbiert wird.

Mittels der in der Steuerungsvorrichtung 70 vorgenommenen und eben beschriebenen Zuordnung der Probe zu einer Klasse können beispielsweise nachfolgende Analyseschritte vereinfacht oder besser geplant werden. Sofern wie beschrieben eine Fehlermeldung ausgegeben wird, kann ein manueller Eingriff ausgelöst werden, um falsche Analysen zu verhindern.

**Patentansprüche**

1.  Verfahren zum Zuordnen einer Blutplasmaprobe zu einer Klasse aus einer vorgegebenen Menge von Klassen, wobei sich die Blutplasmaprobe in einem zumindest teilweise transparenten Gefäß (35) befindet, mit den Schritten:

    - Bestrahlen der Blutplasmaprobe mit Licht (25), dessen spektrale Zusammensetzung zumindest Wellenlängen aus einer vorgegebenen Menge von Wellenlängen aufweist,
    - Bilden eines wellenlängenspezifischen Messwertes für eine jeweilige Wellenlänge aus der Menge von Wellenlängen, wobei ein jeweiliger Messwert von einer durch die Blutplasmaprobe und das Gefäß (35) transmittierten Lichtleistung bei der jeweiligen Wellenlänge abhängig ist, und
    - Zuordnen der Blutplasmaprobe zu einer Klasse in Abhängigkeit von den wellenlängenspezifischen Messwerten,
    - wobei die vorgegebene Menge von Klassen eine hämolytische Klasse aufweist,
    - die Menge von Wellenlängen eine erste hämolytisch-Wellenlänge im Bereich von 535 nm bis 547 nm, bevorzugt 541 nm, aufweist und eine zweite hämolytisch-Wellenlänge im Bereich von 510 nm bis 520 nm, bevorzugt 515 nm, aufweist und
    - die Blutplasmaprobe der hämolytischen Klasse zugeordnet wird, wenn ein Verhältnis des Messwerts bei der ersten hämolytisch-Wellenlänge und des Messwerts bei der zweiten hämolytisch-Wellenlänge kleiner als ein erster relativer hämolytisch-Schwellenwert ist.

**2.** Verfahren nach Anspruch 1, **dadurch gekennzeichnet, dass**

- die vorgegebene Menge von Klassen eine lipämische Klasse aufweist,
- die Menge von Wellenlängen eine lipämisch-Wellenlänge im Bereich von 610 nm bis 700 nm, bevorzugt 650 nm oder 685 nm, aufweist und
- die Blutplasmaprobe der lipämischen Klasse zugeordnet wird, wenn der Messwert bei der lipämisch-Wellenlänge kleiner als ein lipämisch-Schwellenwert ist.

**3.** Verfahren nach einem der vorhergehenden Ansprüche, **dadurch gekennzeichnet, dass**

- die vorgegebene Menge von Klassen eine lipämische Klasse aufweist, und
- ein Streuungs-Messwert gebildet wird, der von einer von der Blutplasmaprobe gestreuten Lichtleistung abhängig ist, wobei die Blutplasmaprobe der lipämischen Klasse zugeordnet wird, wenn der Streuungs-Messwert größer als ein lipämisch-Streuungsschwellenwert ist.

**4.** Verfahren nach einem der vorhergehenden Ansprüche, **dadurch gekennzeichnet, dass**

- die Blutplasmaprobe der hämolytischen Klasse zugeordnet wird, wenn der Messwert bei der ersten hämolytisch-Wellenlänge kleiner als ein absoluter hämolytisch-Schwellenwert ist und wenn die Blutplasmaprobe keiner lipämischen Klasse zugeordnet wird.

**5.** Verfahren nach einem der vorhergehenden Ansprüche, **dadurch gekennzeichnet, dass**

- die Menge von Wellenlängen eine dritte hämolytisch-Wellenlänge im Bereich von 610 nm bis 700 nm, bevorzugt 650 nm oder 685 nm, aufweist und
- die Blutplasmaprobe der hämolytischen Klasse zugeordnet wird, wenn ein Verhältnis des Messwerts bei der ersten hämolytisch-Wellenlänge und des Messwerts bei der dritten hämolytisch-Wellenlänge kleiner als ein zweiter relativer hämolytisch-Schwellenwert ist.

**6.** Verfahren nach einem der vorhergehenden Ansprüche, **dadurch gekennzeichnet, dass**

- die vorgegebene Menge von Klassen eine ikterische Klasse aufweist,
- die Menge von Wellenlängen eine erste ikterisch-Wellenlänge im Bereich von 450 nm bis 485 nm, bevorzugt 470 nm, aufweist und
- die Blutplasmaprobe der ikterischen Klasse zugeordnet wird, wenn der Messwert bei der ersten ikterisch-Wellenlänge kleiner als ein absoluter ikterisch-Schwellenwert ist und wenn die Blutplasmaprobe keiner lipämischen Klasse zugeordnet wird und keiner hämolytischen Klasse zugeordnet wird.

**7.** Verfahren nach einem der vorhergehenden Ansprüche, **dadurch gekennzeichnet, dass**

- die vorgegebene Menge von Klassen eine ikterische Klasse aufweist,
- die Menge von Wellenlängen eine erste ikterisch-Wellenlänge im Bereich von 450 nm bis 485 nm, bevorzugt 470 nm, aufweist und eine zweite ikterisch-Wellenlänge im Bereich von 510 nm bis 520 nm, bevorzugt 515 nm, aufweist und
- die Blutplasmaprobe der ikterischen Klasse zugeordnet wird, wenn ein Verhältnis des Messwerts bei der ersten ikterisch-Wellenlänge und des Messwerts bei der zweiten ikterisch-Wellenlänge kleiner als ein erster relativer ikterisch-Schwellenwert ist.

**8.** Verfahren nach einem der vorhergehenden Ansprüche, **dadurch gekennzeichnet, dass**

- die vorgegebene Menge von Klassen eine gute Klasse aufweist, und
- die Blutplasmaprobe der guten Klasse zugeordnet wird, wenn die Blutplasmaprobe keiner lipämischen Klasse zugeordnet wird, keiner hämolytischen Klasse zugeordnet wird und keiner ikterischen Klasse zugeordnet wird.

**9.** Verfahren nach einem der vorhergehenden Ansprüche, **dadurch gekennzeichnet, dass**

- eine Fehlermeldung ausgegeben wird, wenn die Blutplasmaprobe zumindest zwei unterschiedlichen Klassen zugeordnet wird.

**10.** Verfahren nach einem der vorhergehenden Ansprüche, **gekennzeichnet durch** den Schritt

- Erkennen, ob und in welcher Lage sich ein Etikett auf dem Gefäß befindet, wobei abhängig davon eine Anzahl von Schwellenwerten verändert wird.

**11.** Vorrichtung (10) zum Zuordnen einer Blutplasmaprobe, die sich in einem zumindest teilweise transparenten Gefäß (35) befindet, zu einer Klasse aus einer vorgegebenen Menge von Klassen, wobei die Vorrichtung zum Durchführen des Verfahrens nach einem der vorhergehenden Ansprüche ausgebildet ist und aufweist:

- eine Lichtquelle (20), die Licht, dessen spektrale Zusammensetzung zumindest Wellenlängen aus der gemäß dem Verfahren vorgegebenen Menge von Wellenlängen aufweist, auf das Gefäß (35) abgibt,
- eine Detektoranordnung (65), die durch die Blutplasmaprobe und das Gefäß (35) transmittiertes Licht aufnimmt und darin für jede der Wellenlängen aus der Menge von Wellenlängen einen von der transmittierten Lichtleistung abhängigen Messwert misst, und
- eine Steuerungsvorrichtung (70), die mit der Detektoranordnung (65) zur Erfassung der gemessenen Messwerte verbunden ist und das Verfahren nach einem der vorhergehenden Ansprüche ausführt.

12. Vorrichtung (10) nach Anspruch 11, **dadurch gekennzeichnet, dass**

- die Detektoranordnung (65) für jede Wellenlänge aus der Menge von Wellenlängen einen Detektor (61, 62, 63, 64), einen dem Detektor (61, 62, 63, 64) vorgeschalteten Filter (51, 52, 53, 54), und eine lichtleitende Faser (41, 42, 43, 44) zur Aufnahme von durch die Probe und das Gefäß (35) transmittiertem Licht aufweist.

13. Vorrichtung (10) nach Anspruch 11 oder 12, **gekennzeichnet durch**

- einen weiteren Detektor (60), der von der Blutplasmaprobe relativ zur geradlinigen Ausbreitung seitlich gestreutes Licht, bevorzugt mit einem Winkel von 90°, aufnimmt und einen von der gestreuten Lichtleistung abhängigen Messwert misst, wobei die Steuerungsvorrichtung (70) mit dem weiteren Detektor (60) zur Erfassung des gemessenen Messwerts verbunden ist.

**Claims**

1. Method for assigning a blood plasma sample to a class from a predetermined set of classes, the blood plasma sample being contained in an at least partially transparent vessel (35), the method comprising the steps of:

- exposing the blood plasma sample to light (25), the spectral composition of which comprises at least wavelengths from a predetermined set of wavelengths,
- forming a wavelength-specific measurement value for a respective wavelength from the set of wavelengths, a respective measurement value being dependent on a light power transmitted through the blood plasma sample and the vessel (35) at the respective wavelength, and
- assigning the blood plasma sample to a class as a function of the wavelength-specific measurement values,
- wherein the predetermined set of classes comprises a hemolytic class,
- the set of wavelengths comprises a first hemolytic wavelength in the range of from 535 nm to 547 nm, preferably 541 nm, and a second hemolytic wavelength in the range of from 510 nm to 520 nm, preferably 515 nm, and
- the blood plasma sample is assigned to the hemolytic class if a ratio of the measurement value at the first hemolytic wavelength and the measurement value at the second hemolytic wavelength is less than a first relative hemolytic threshold value.

2. Method according to claim 1, **characterized in that**

- the predetermined set of classes comprises a lipemic class,
- the set of wavelengths comprises a lipemic wavelength in the range of from 610 nm to 700 nm, preferably 650 nm or 685 nm, and
- the blood plasma sample is assigned to the lipemic class if the measurement value at the lipemic wavelength is less than a lipemic threshold value.

3. Method according to any of the preceding claims, **characterized in that**

- the predetermined set of classes comprises a lipemic class, and
- a scattering measurement value is formed, which is dependent on a light power scattered by the blood plasma sample, the blood plasma sample being assigned to the lipemic class if the scattering measurement value is greater than a lipemic scattering threshold value.

4. Method according to any of the preceding claims, **characterized in that**

- the blood plasma sample is assigned to the hemolytic class if the measurement value at the first hemolytic wavelength is less than an absolute hemolytic threshold value and if the blood plasma sample is not assigned to a lipemic class.

5. Method according to any of the preceding claims, **characterized in that**

- the set of wavelengths comprises a third hemolytic wavelength in the range of from 610 nm to 700 nm, preferably 650 nm or 685 nm, and
- the blood plasma sample is assigned to the hemolytic class if a ratio of the measurement value at the first hemolytic wavelength and the measurement value at the third hemolytic wavelength is less than a second relative hemolytic threshold value.

6. Method according to any of the preceding claims, **characterized in that**

- the predetermined set of classes comprises an icteric class,
- the set of wavelengths comprises a first icteric wavelength in the range of from 450 nm to 485 nm, preferably 470 nm, and
- the blood plasma sample is assigned to the icteric class if the measurement value at the first icteric wavelength is less than an absolute icteric threshold value and if the blood plasma sample is not assigned to a lipemic class and is not assigned to a hemolytic class.

7. Method according to any of the preceding claims, **characterized in that**

- the predetermined set of classes comprises an icteric class,
- the set of wavelengths comprises a first icteric wavelength in the range of from 450 nm to 485 nm, preferably 470 nm, and a second icteric wavelength in the range of from 510 nm to 520 nm, preferably 515 nm, and
- the blood plasma sample is assigned to the icteric class if a ratio of the measurement value at the first icteric wavelength and the measurement value at the second icteric wavelength is less than a first relative icteric threshold value.

8. Method according to any of the preceding claims, **characterized in that**

- the predetermined set of classes comprises a good class, and
- the blood plasma sample is assigned to the good class if the blood plasma sample is not assigned to a lipemic class, is not assigned to a hemolytic class and is not assigned to an icteric class.

9. Method according to any of the preceding claims, **characterized in that**

- an error message is emitted if the blood plasma sample is assigned to at least two different classes.

10. Method according to any of the preceding claims, **characterized by** the step of

- detecting whether, and at what position, there is a label on the vessel, a number of threshold values being modified as a function thereof.

11. Device (10) for assigning a blood plasma sample, which is contained in an at least partially transparent vessel (35), to a class from a predetermined set of classes, the device being configured in order to carry out the method according to any of the preceding claims and comprising:

- a light source (20) which emits light, the spectral composition of which comprises at least wavelengths from the predetermined set of wavelengths according to the method, onto the vessel (35),
- a detector arrangement (65), which receives light transmitted through the blood plasma sample and the vessel (35) and measures therein a measurement value dependent on the transmitted light power for each of the wavelengths from the set of wavelengths, and
- a control device (70), which is connected to the detector arrangement (65) in order to record the measured measurement values and carries out the method according to any of the preceding claims.

12. Device (10) according to claim 11, **characterized in that**

- the detector arrangement (65) comprises, for each wavelength from the set of wavelengths, a detector (61, 62, 63, 64), a filter (51, 52, 53, 54) arranged upstream of the detector (61, 62, 63, 64), and a light-guiding fiber (41, 42, 43, 44) for receiving light transmitted through the sample and the vessel (35).

13. Device (10) according to claim 11 or 12, **characterized by**

- a further detector (60), which receives light scattered laterally, preferably with an angle of 90°, by the blood plasma sample relative to propagation in a straight line, and measures a measurement value dependent on the scattered light power, the control device (70) being connected to the further detector (60) in order to record the measured measurement value.

**Revendications**

1. Procédé d'association d'un échantillon de plasma sanguin à une classe appartenant à un ensemble prédéterminé de classes, dans lequel l'échantillon de plasma sanguin se trouve dans un récipient (35) au moins partiellement transparent, comportant les étapes consistant à :

- exposer l'échantillon de plasma sanguin à une lumière (25) dont la composition spectrale comporte au moins des longueurs d'onde appartenant à un ensemble prédéterminé de longueurs d'onde,
- établir une valeur de mesure spécifique de la longueur d'onde pour une longueur d'onde respective appartenant à l'ensemble de longueurs d'onde, dans lequel une valeur de mesure respective dépend d'une puissance lumineuse transmise à travers l'échantillon de plasma sanguin et le récipient (35) à la longueur d'onde respective, et
- associer l'échantillon de plasma sanguin à une classe en fonction des valeurs de mesure spécifiques de la longueur d'onde,
- dans lequel l'ensemble prédéterminé de classes comprend une classe hémolytique,
- l'ensemble de longueurs d'onde comporte une première longueur d'onde hémolytique comprise dans la plage de 535 nm à 547 nm, de préférence 541 nm, et une deuxième longueur d'onde hémolytique dans la plage de 510 nm à 520 nm, de préférence 515 nm, et
- l'échantillon de plasma sanguin est associé à la classe hémolytique lorsqu'un rapport de la valeur de mesure à la première longueur d'onde hémolytique à la valeur de mesure à la deuxième longueur d'onde hémolytique est inférieur à une première valeur de seuil hémolytique relative.

2. Procédé selon la revendication 1, **caractérisé en ce que**

- l'ensemble prédéterminé de classes comporte une classe lipémique,
- l'ensemble de longueurs d'onde comporte une longueur d'onde lipémique dans la plage de 610 nm à 700 nm, de préférence 650 nm ou 685 nm, et
- l'échantillon de plasma sanguin est associé à la classe lipémique lorsque la valeur de mesure à la longueur d'onde lipémique est inférieure à une valeur de seuil lipémique.

3. Procédé selon l'une quelconque des revendications précédentes, **caractérisé en ce que**

- l'ensemble prédéterminé de classes comporte une classe lipémique, et
- une valeur de mesure de diffusion qui dépend d'une puissance lumineuse diffusée par l'échantillon de plasma sanguin est établie, dans lequel l'échantillon de plasma sanguin est associé à la classe lipémique lorsque la valeur de mesure de diffusion est supérieure à une valeur de seuil de diffusion lipémique.

4. Procédé selon l'une quelconque des revendications précédentes, **caractérisé en ce que**

- l'échantillon de plasma sanguin est associé à la classe hémolytique lorsque la valeur de mesure à la première longueur d'onde hémolytique est inférieure à une valeur de seuil hémolytique absolue et lorsque l'échantillon de plasma sanguin n'est associé à aucune classe lipémique.

5. Procédé selon l'une quelconque des revendications précédentes, **caractérisé en ce que**

- l'ensemble de longueurs d'onde comporte une troisième longueur d'onde hémolytique dans la plage de 610 nm à 700 nm, de préférence 650 nm ou 685 nm, et
- l'échantillon de plasma sanguin est associé à la classe hémolytique lorsqu'un rapport de la valeur de mesure à la première longueur d'onde hémolytique à la valeur de mesure à la troisième longueur d'onde hémolytique est inférieur à une deuxième valeur de seuil hémolytique relative.

6. Procédé selon l'une quelconque des revendications précédentes, **caractérisé en ce que**

- l'ensemble prédéterminé de classes comporte une classe ictérique,
- l'ensemble de longueurs d'onde comporte une première longueur d'onde ictérique dans la plage de 450 nm à 485 nm, de préférence 470 nm, et
- l'échantillon de plasma sanguin est associé à la classe ictérique lorsque la valeur de mesure à la première longueur d'onde ictérique est inférieure à une valeur de seuil ictérique absolue et lorsque l'échantillon de plasma sanguin n'est associé à aucune classe lipémique et n'est associée à aucune classe hémolytique.

7. Procédé selon l'une quelconque des revendications précédentes, **caractérisé en ce que**

- l'ensemble prédéterminé de classes comporte une classe ictérique,
- l'ensemble de longueurs d'onde comporte une première longueur d'onde ictérique dans la plage de 450 nm à 485 nm, de préférence 470 nm, et comporte une deuxième longueur d'onde ictérique dans la plage de 510 nm à 520 nm, de préférence 515 nm, et

- l'échantillon de plasma sanguin est associé à la classe ictérique lorsqu'un rapport de la valeur de mesure à la première longueur d'onde ictérique à la valeur de mesure à la deuxième longueur d'onde ictérique est inférieur à une première valeur de seuil ictérique relative.

8. Procédé selon l'une quelconque des revendications précédentes, **caractérisé en ce que**

    - l'ensemble prédéterminé de classes comporte une classe correcte, et
    - l'échantillon de plasma sanguin est associé à la classe correcte lorsque l'échantillon de plasma sanguin n'est associé à aucune classe lipémique, n'est associé à aucune classe hémolytique et n'est associé à aucune classe ictérique.

9. Procédé selon l'une quelconque des revendications précédentes, **caractérisé**

    - **en ce qu'**un message d'erreur est émis lorsque l'échantillon de plasma sanguin est associé à au moins deux classes différentes.

10. Procédé selon l'une quelconque des revendications précédentes, **caractérisé par** l'étape consistant à

    - détecter si une étiquette se trouve sur le récipient et à quelle position, dans lequel un nombre de valeurs de seuil est modifié en fonction de ladite détection.

11. Dispositif (10) d'association d'un échantillon de plasma sanguin se trouvant dans un récipient (35) au moins partiellement transparent à une classe appartenant à un ensemble prédéterminé de classes, dans lequel le dispositif est conçu pour mettre en oeuvre le procédé selon l'une quelconque des revendications précédentes et comporte :

    - une source lumineuse (20) qui émet vers le récipient (35) une lumière dont la composition spectrale comporte au moins des longueurs d'onde appartenant à l'ensemble prédéterminé de longueurs d'onde conformément au procédé,
    - un système détecteur (65) qui capte la lumière transmise à travers l'échantillon de plasma sanguin et le récipient (35) et qui mesure dans celui-ci, pour chacune des longueurs d'onde appartenant à l'ensemble de longueurs d'onde, une valeur de mesure dépendant de la puissance lumineuse transmise, et
    - un dispositif de commande (70) qui est relié au système détecteur (65) pour détecter les valeurs de mesure mesurées et qui met en oeuvre le procédé selon l'une quelconque des revendications précédentes.

12. Dispositif (10) selon la revendication 11, **caractérisé en ce que**

    - l'ensemble détecteur (65) comporte, pour chaque longueur d'onde appartenant à l'ensemble de longueurs d'onde, un détecteur (61, 62, 63, 64), un filtre (51, 52, 53, 54) disposé en amont du détecteur (61, 62, 63, 64) et une fibre optique (41, 42, 43, 44) destinée à capter la lumière transmise à travers l'échantillon et le récipient (35).

13. Dispositif (10) selon la revendication 11 ou 12, **caractérisé**

    - **en ce qu'**un autre détecteur (60), qui capte la lumière diffusée latéralement par l'échantillon de plasma sanguin par rapport à la propagation linéaire, de préférence à un angle de 90°, et qui mesure une valeur de mesure dépendant de la puissance lumineuse diffusée, dans lequel le dispositif de commande (70) est relié à l'autre détecteur (60) pour détecter la valeur de mesure mesurée.

Fig. 1

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2549264 A